# EUROPEAN PATENT SPECIFICATION

(11) **EP 2 794 720 B1**
(45) Date of publication and mention of the grant of the patent: **02.08.2017**
(21) Application number: 12805449.1
(22) Date of filing: 18.12.2012
(51) Int. Cl.: C08G 65/00

(54) **PROCESS FOR THE MANUFACTURE OF (PER)FLUOROPOLYETHERS WITH AROMATIC END GROUPS**
VERFAHREN ZUR HERSTELLUNG VON (PER)FLUORPOLYETHERN MIT AROMATISCHEN ENDGRUPPEN
PROCÉDÉ DE FABRICATION DE (PER)FLUOROPOLYÉTHERS AVEC GROUPES TERMINAUX AROMATIQUES

(30) Priority: 22.12.2011 EP 11195231
(43) Date of publication of application: 29.10.2014
(73) Proprietor: Solvay Specialty Polymers Italy S.p.A., 20021 Bollate (MI) (IT)
(72) Inventor: TONELLI, Claudio Adolfo Pietro, I-23877 Paderno D'adda (Lecco) (IT); AVATANEO, Marco, I-20030 Senago (Milano) (IT); MARCHIONNI, Giuseppe, I-20133 Milano (IT)
(74) Representative: Benvenuti, Federica
(86) International application number: PCT/EP2012/076023
(87) International publication number: WO 2013/092632

(56) References cited:
- EP-A1- 2 100 909
- WO-A1-2009/010533
- WO-A1-2009/019243
- WO-A1-2010/063745
- US-A- 4 454 349
- US-A1- 2006 293 534

## Description

### Technical Field

The present invention relates to a process for preparing PFPE derivatives having aromatic end groups. Such PFPE derivatives and to their use as additives for fluorinated oils and greases are also herein described.

### Background Art

(Per)fluoropolyether (PFPE) oils and greases are endowed with high chemical and thermal stability and they have long since been used as lubricants; however, under drastic conditions, for example at high temperatures and for prolonged times, in the presence of Lewis acids and/or metals, especially also in the presence of water, degradation occurs, with formation of volatile fractions, which may lead to complete lubricant loss. For this reason, PFPE oils and greases often contain additives able to increase their thermal and chemical stability. For instance, EP 1479753 A (SOLVAY SOLEXIS SPA) discloses (per)fluoropolyether additives formed of (per)fluoropolyether chains and end groups having an optionally substituted pyridine structure; US 4681693 B (AUSIMONT SPA) discloses stabilizers with arylphosphinic structure which contain perfluoropolyether chains bonded to the benzene ring of the phosphine through a non-perfluorinated bridge; EP 1354932 A (SOLVAY SOLEXIS SPA) discloses perfluoropolyether derivatives comprising a perfluoropolyether chain having two chain ends, each chain end bearing an optionally substituted phenyl ring linked to the chain through a -CH₂O- or a -C(O)O- group. It has nonetheless been observed that these additives undergo decomposition if particularly harsh conditions, e.g. temperatures above 300°C, especially in the presence of water or humidity, are maintained for prolonged times.

US 2006293534 (DU PONT DE NEMOURS AND CO.) discloses a process for the preparation of a fluoropolyether- or fluoroalkyl- substituted aryl pnictogen comprising contacting a compound selected from the group consisting of fluoropolyether or fluoroalkyl primary halide, with a triaryl phosphine, triaryl arsine, or triaryl stibine, triaryl phosphine oxide, triaryl arsine oxide, or triaryl stibine oxide in the presence of a radical initiator, to produce the corresponding mono-substituted, di-substituted, trisubstituted, or a combination of two or more thereof, fluoropolyether- or fluoroalkyl-substituted aryl phosphine oxide, aryl arsine oxide or aryl stibine oxide and optionally reducing the resulting oxide with a reducing agent. In particular, the document discloses aryl pnictogen compounds wherein the ayl moiety bears perfluoropolyether chains of formula: F(CF(CF₃)CF₂O)_{z}CF(CF₃)CF₂-.

US 4011267 (TAMBORSKI ET AL) and US 4454349 (TAMBORSKI ET AL) disclose perfluoropolyether substituted arylphosphines useful as anticorrosion and antioxidation additives for perfluorinated fluids, wherein a perfluoropolyether chain is linked to the arylphosphine moiety through a - CF₂- bridging group. However, such compounds are prepared through processes that are not suitable for industrial scale, because they involve the use of organometallic compounds, e.g. Grignard reagents, or toxic and corrosive fluorinating reagents, namely SF₄ and HF, for obtaining the - CF₂- bridging group.

(Per)fluoropolyether peroxides are known as intermediates in the preparation of (per)fluoropolyether derivatives. For instance, they have been disclosed in EP 2176162 A (SOLVAY SOLEXIS SPA) as intermediates for the preparation of modified carbonaceous materials comprising a (per)fluoropolyether chain chemically bound to the material's surface. The modified carbonaceous materials are endowed with improved surface properties, e.g. hydro- and oleo-repellence. (Per)fluoropolyether peroxides have also been disclosed in EP 2170968 A (SOLVAY SOLEXIS SPA) as intermediates for the preparation of block polymers comprising non-fluorinated aromatic polymers segments and (per)fluoropolyether segments; said compounds are endowed with oil- and water-repellency properties and are said to be stable to temperatures as high as 100°C. They are also said to be suitable for the preparation of mechanical parts, components of power stations, structural parts of vehicles or aircraft, in the motor vehicles and transport industry and the chemical industry. This prior art does not give any hint or suggestion regarding the preparation of (per)fluoropolyether derivatives to be used as lubricant additives and having high chemical and thermal stability under particularly harsh conditions.

EP 2100909 A (SOLVAY SOLEXIS SPA) 9/10/2009 discloses a process for the addition of at least one PFPE peroxide comprising at least one peroxidic moiety and at least one PFPE chain R_{f} onto a per(halo)fluorinated aromatic compound, so as to yield an addition compound comprising at least one perfluorinated non aromatic cyclic moiety having at least two substituents comprising a R_{f} chain and, optionally, conjugated or non conjugated double bonds. Example 6 of this application discloses the preparation of an addition compound of formula:

T-O-[R_{f}-(CF₂)_{y}-W-(O)_{y'}]_{b}-R_{f}-T'

wherein:
W is divalent unsaturated cyclic unit of formula -C₆F₅N-;
y and y' are O or 1,
R_{f} are perfluoropolyether blocks of formula: -

   [(CF₂CF₂O)ₘ(CF₂O)ₙ(CF₂CF₂CF₂O)ₚ(CF₂CF₂CF₂CF₂O)_{q}];
m/n=0.96, p/n=0.008 and q/n=0.0053;
b is 6;
Mn = 26,000
T and T' are selected from -CF₃, -CF₂CF₂Cl, -CF₂COF.

The addition compounds disclosed in EP 2100909 A are disclosed as additives in (per)fluorinated heat exchanges fluids and lubricants and as raw materials for manufacturing cured gums and grafted copolymers.

WO 2009/019243 (SOLVAY SOLEXIS SPA) 2/12/2009 discloses (per)fluoropolyethers comprising at least one (per)fluoropolyoxyalkylene chain (chain R_{f}) comprising at least one recurring unit (R2) having formula: -CF₂ CF(CF₂OSO₂F)- [poly(fluorosulfate) PFPE]. These compounds can be prepared by means of a process which comprises reacting a (per)fluoropolyether peroxide (peroxidic PFPE) with CF₂=CFCF₂OSO₂F (FAFS). These compounds are useful for the preparation of PFPE derivatives.

The need was thus felt of providing (per)fluoropolyether additives suitable as stabilizers for (per)fluorinated oils and greases endowed with improved resistance to temperatures as high as 300°C, even in the presence of metals, Lewis acids and water or humidity and that could be prepared through a convenient and industrially applicable process.

### Summary of invention

It has now been found that (per)fluoropolyether compounds endowed with the aforementioned properties can be conveniently prepared through a process which comprises the reaction of a (per)fluoropolyether peroxide with an optionally substituted aromatic or heteroaromatic compound.

Thus, in a first aspect, the present invention relates to a process which comprises the reaction of a (per)fluoropolyether peroxide with an optionally substituted aromatic or heteroaromatic compound to provide a (per)fluoropolyether compound comprising one (per)fluoropolyether chain having two chain ends, wherein one or both chain ends is a -CF₂- group covalently bound to the aromatic or heteroaromatic group through a carbon-carbon bond,
wherein said aromatic compound is benzene, biphenyl, phenantrene, anthracene, benzoic acid, fluorobenzene, bromobenzene, trifluoromethylbenzene, nitrobenzene, methyl- or ethyl-benzoate, dimethylterephthalate, and pirocathecol;
said aromatic or heteroaromatic compound can bear one or more substituents, which may be the same or different from one another, said substituents on the aromatic compound being halogen, (per)haloalkyl, nitro, amino, amido, nitrile, ester groups and phosphorous- and sulphur-containing groups.

Typically, the process of the invention comprises the reaction of a (per)fluoropolyether peroxide with an optionally substituted aromatic or heteroaromatic compound to provide an addition compound [compound (A)] comprising a (per)fluoropolyether chain having two chain ends, wherein one or one or both chain ends is a -CF₂- group covalently bound to the aromatic or heteroaromatic group through a carbon-carbon bond. For the purpose of the present description, the expression "addition compound" is intended to exclude polycondensation polymers comprising recurring fluorinated blocks and recurring aromatic blocks. In other words, if both chain ends are both -CF₂- groups covalently bound to an aromatic or heteroaromatic group through a carbon-carbon bond, the aromatic or heteroaromatic groups are not substituted with (per)fluoropolyether chains. According to a preferred embodiment, addition compound (A) comprises one single (per)fluoropolyether chain having two chain ends, wherein one or one or both chain ends is a -CF₂- group covalently bound to the aromatic or heteroaromatic group through a carbon-carbon bond.

For the purposes of the present invention, the (per)fluoropolyether peroxide [peroxide (P)] is a (per)fluoropolyether peroxide comprising at least one peroxidic moiety comprised between sp³ carbon atoms and at least one fully or partially fluorinated polyoxyalkene chain (chain R_{f}), i.e. a fluorocarbon segment comprising ether linkages in the main chain.

The expression "at least one peroxide moiety" is understood to mean that the peroxide (P) comprises one or more than one peroxide moiety. Herein below, the expression "peroxide (P)" shall be understood both in the singular and in the plural.

The peroxide moiety of the peroxide (P) is comprised between sp³ hybridized carbon atoms: thanks to this layout, thermal and storage stability of the peroxide (P) is advantageously increased over, notably, that of perfluoroalkanoyl peroxides, wherein the -OO- group is bound to -C(O)-sp² hybridized carbon atoms.

The fluoropolyoxyalkene chain (R_{f}) of the peroxide (P) is preferably a chain comprising repeating units R°, statistically distributed along the chain, said repeating units being chosen among the group consisting of:
(i) (CFXO), wherein X is F or CF₃;
(ii) (CF₂CF₂O);
(iii) (C₃F₆O) units of formula (CF₂CF₂CF₂O), (CF₂CF(CF₃)O) and (CF(CF₃)CF₂O);
(iv) (CF₂CF₂CF₂CF₂O);
(v) (CR₅R₆CF₂CF₂O), wherein R₅ and R₆, which may be identical or different, are chosen from H, Cl and perfluoroalkyl containing from 1 to 4 carbon atoms;
(vi) -(CF₂)ⱼ-CFZ-O- wherein j is an integer from 0 to 3 and Z is a group of general formula -OR_{f}'T, wherein R_{f}' is a fluoropolyoxyalkene chain comprising a number of repeating units from 0 to 10, said repeating units being chosen among the followings : -CFXO-, -CF₂CFXO-, -CF₂CF₂CF₂O-, -CF₂CF₂CF₂CF₂O-, wherein X is independently F or CF₃ and T is a C₁-C₃ perfluoroalkyl group.

Preferably, the peroxide moieties of peroxide (P) are randomly distributed in the perfluoropolyoxyalkylene chain.

Peroxide (P) preferably complies with formula (I) here below:

A-O-(CFXO)_{c1}(CF₂CF₂O)_{c2}(C₃F₆O)_{c3}(CF₂CF₂CF₂CF₂O)_{c4}(O)ₚ-B formula (I)

wherein:
- A and B, equal to or different from each other, are independently selected from -CF₃, -CF₂-CF₃, -CF₂Cl, -CF₂CF₂Cl, -CF(CF₃)COF, -CF₂-COF, -COF;
- X is F or CF₃;
- c1, c2 , c3, and c4, equal or different from each other, are independently integers ≥0 and p is an integer > 0, wherein c1, c2 , c3, c4 and p are selected in such a way that the average number molecular weight of the peroxide ranges from 300 to 150,000 and p is selected in such as way as the content of peroxide groups -O-O,- or oxidizing power (OP) is between 0.02 % and 4 %, preferably between 0.2 % and 4.0 % and more preferably between 0.5 % and 3 %. For the purposes of the present description, the " oxidizing power" is expressed as grams of active oxygen per 100 grams of peroxide (P). Quantitative methods for measuring the oxidizing power are disclosed, for example, in BYNYARD, W. C., et al. Perfluoropolyether Synthesis in Liquid Carbon Dioxide by Hexafluoroprolylene Photooxidation. Macromolecules. 1999, no.32, p.8224-8226. and in GUARDA, P.A., et al. Peroxidic perfluoropolyether from tetrafluoroethylene oxidation: microstructural analysis by NMR spectroscopy and mechanistic considerations. Journal of Fluorine Chemistry. 2005, no.126, p.685-697.

The peroxidic PFPEs can be prepared, for example, by photoassisted polymerization of tetrafluoroethylene (TFE) and/or hexafluoropropene (HFP), in the presence of oxygen according to the teachings of US 3442942 (MONTEDISON SPA), US 3650928 (MONTEDISON SPA) and US 3665041 (MONTEDISON SPA).

The peroxidic PFPEs containing -(CF₂)ⱼ-CFZ-O- units can be prepared, for example, according to what described in US 5144092 (AUSIMONT SRL) by polymerization, in the presence of oxygen and UV, of one or more (per)fluoroalkylvinylethers of formula CF₂=CFOXa wherein Xa is one or more groups (R'O)ₘR", equal to or different from each other, wherein m = 0-6, R' is selected from the groups -CF₂-, -CF₂CF₂-, -CF₂CF(CF₃)-, R" is selected from C₁-C₁₀ linear perfluoroalkyl, or C₃-C₁₀ branched perfluoroalkyl, or C₃-C₁₀ cyclic perfluoroalkyl, in the presence of a solvent and at a temperature not higher than 50°C. This process can be carried out also in the presence of TFE and/or HFP. See furthermore, for example, EP 1454938 A (SOLVAY SOLEXIS SPA), EP 1524287 A (SOLVAY SOLEXIS SPA).

Preferably, peroxide (P) is selected from the following groups:
(A)

   Xₒ-O(CF₂CF₂O)ᵣ(CF₂O)ₛ(O)ₚ-Xₒ' formula (II-A)

   wherein
   Xₒ and Xₒ', equal to or different from each other, are -CF₂Cl, -CF₂CF₂Cl, -CF₃, -CF₂CF₃, -CF₂COF, -COF;
   r and s are integers ≥ 0 and p is an integer >0, selected in such a way that the number average molecular weight of the peroxide is between 400 and 150,000, preferably between 500 and 80 000 and p is selected in such a way that the oxidizing power is in the above-defined range.

Preferably, both r and s are different from zero and the r/s ratio is comprised between 0.1 and 10.

Peroxides (P) complying with formula (IIA) here above can be prepared by tetrafluoroethylene oxypolymerization, notably following the teachings of US 3715378 (MONTEDISON SPA), US 4451646 (MONTEDISON SPA), US 5258110 (AUSIMONT SRL), US 5744651 (AUSIMONT SPA) ;
(B)

   X₁-O(CF₂CF₂O)ᵣ(CF₂O)ₛ(CF(CF₃)O)ᵤ(CF₂CF(CF₃)O)ᵥ(O)ₚ-X_{1'} formula (II-B)

   wherein:
   X₁ and X_{1'}, equal to or different from each other, are -CF₂Cl, -CF₂CF₂Cl,-CF₂CF₃, -CF₃, -C₃F₇, -CF(CF₃)COF, -COF, -CF₂COF, -CF₂C(O)CF₃,-COF;
   r, s, u, v are integers ≥ 0 and p is an integer >0, selected in such a way that the number average molecular weight of the peroxide is between 500 and 150 000, preferably between 700 and 80 000 and p is selected in such a way that the oxidizing power is in the above-defined range.

Preferably r, s, u, v are all integers > 0 and the v/(r+s+u) ratio is < 1.

Peroxides (P) complying with formula (II-B) here above can be prepared by oxypolymerization of tetrafluoroethylene and hexafluoropropylene, notably following the teachings of US 5000830 (AUSIMONT SRL) and US 3847978 (MONTEDISON SPA);
(C)

   X₂-O(CF₂CF₂O)ᵣ(CF₂O)ₛ(CF₂(CF₂)_{w}CF₂O)ₖ(O)ₚ-X_{2'} formula (II-C)

   wherein:
   X₂ and X_{2'}, equal to or different from each other, are -CF₂COF, -COF;
   w is 1 or 2;
   r, s, and k are integers ≥ 0 and p is an integer >0, selected in such a way that that the number average molecular weight is between 500 and 100,000 and p is selected in such a way that the oxidizing power is in the above-defined range.

Preferably r, s and k are all > 0, the r/s ratio being typically between 0.2 and 10, and the ratio k/(r+s) being generally lower than 0.05. Peroxides (P) complying with formula (II-C) above can be prepared following teachings of US 2005192413 (SOLVAY SOLEXIS SPA).

The expression "optionally substituted aromatic or heteroaromatic compound" is intended to mean any an aromatic or heteroaromatic compound optionally containing substituent groups which do not undergo radical hydrogen removal; for instance, aromatic or heteroaromatic compounds substituted with alkyl groups containing primary, secondary or tertiary carbon atoms directly linked to the aromatic ring or to a heteroatom like nitrogen, oxygen and sulphur are usually not compatible with the process of the invention. A skilled person will be able to determine other non - compatible compounds on the basis of the common general knowledge.

Examples of aromatic compounds are C₆-C₁₆ aromatic compounds, while examples of heteroaromatic compounds are C₅-C₁₆ aromatic compounds containing one or more heteroatoms independently selected from N, O and S; the aromatic or heteroaromatic compounds may also be covalently bound to or condensed with one ore more substituted aromatic or heteroaromatic compounds as defined above.

Examples of preferred aromatic and heteroaromatic compounds according to the present invention are benzene, biphenyl, phenanthrene, anthracene, benzoic acid, fluorobenzene, bromobenzene, trifluoromethylbenzene, nitrobenzene, methyl- or ethyl-benzoate, dimethylterephthalate, and pirocathecol. Bis(trifluoromethyl)acetate can also be used.

Examples of preferred substituents on the aromatic compound are halogen, (per)haloalkyl, nitro, amino, amido, nitrile, ester groups and phosphorus- and sulfur-containing groups. The aromatic or heteroaromatic compound can bear one or more substituents, which may be the same or different from one another.

The process of the invention can be carried out under continuous, semi-continuous or discontinuous conditions.

The selected peroxidic PFPE is typically reacted with the aromatic or the heteroaromatic compound at a temperature selected from -80°C to 300°C, optionally under UV catalysis. In particular, UV catalysis is advantageous when the temperature is lower than 150°C; in the absence of UV catalysis, the preferred temperature range is 70-300°C; more preferably, it is of 100-250°C.

Typically, pressure ranges from ambient pressure to 2000 kPa.

The molar ratio between the peroxide groups in the peroxidic PFPE and the aromatic compound typically ranges from 0.01 to 100, more preferably from 0.1 to 50, still more preferably from 0.5 to 10.

The reaction is typically complete in a time range of from 10 min to 24 hrs, according to the reaction temperature; when the reaction is carried out at 200°C, the reaction time is typically of 8 hrs.

The reaction can be carried out either in bulk or in the presence of a solvent; suitable solvents include (per)fluoropolyethers, fro example Galden^{®} (per)fluoropolyethers, hydrofluoro(poly)ethers, perfluorobutylamine, chlorofluorocarbons (CFCs), fluorocarbons (FCs), hydrochlorofluorocarbons (HCFCs), (per)fluoropolyethers and being preferred. (Per)fluoropolyethers like H-Galden^{®} PFPEs and hydrofluoro(poly)ethers being preferred. In particular, a solvent is preferably used when the peroxide number (PO) of the peroxidic PFPE is higher than 2.5.

Preferably, the reaction mixture of peroxidic PFPE and aromatic or heteroaromatic compound and optional solvent is kept under stirring.

According to one embodiment, the reagents and any solvent are mixed at room temperature, then the temperature is gradually increased to a desired value. According to an alternative embodiment, one of the reagents is brought to the desired reaction temperature, then the other one is gradually added.

The process of the invention allows to obtain PFPE compounds containing a (per)fluoropolyether chain (chain R_{f}) having two chain ends, in which one or both chain ends is a -CF₂- group bound to an optionally substituted aromatic or heteroaromatic moiety through a -carbon-carbon- bond. It has indeed been observed that no ether bond is formed between sp² carbon atoms of the aromatic or heteroaromatic group and the carbon atom of the -CF₂- group upon reaction of the (per)fluoropolyether peroxide and the aromatic or heteroaromatic compound.

These compounds are described herein after.

Typically, the process of the invention leads to (per)fluoropolyether addition compounds (A) as defined above complying with formula (III) below:

(T₁-CF₂O-R_{f}-CF₂)ₙ-T₃ (III)

in which:
- T₁ is either selected from -F, -CF₃, -CF₂Cl, -COF or is the same as T₃;
- R_{f} is a (per)fluoropolyether chain as defined above;
- n is an integer from 1 to 10, preferably from 1 to 5, more preferably from 1, 2 and 3; most preferably, n is 1;
- T₃ is an optionally substituted aromatic or heteroaromatic moiety,
In particular, if T3 is a substituted aromatic or heteroaromatic moiety, the substituent is not a (per)fluoropolyether chain; furthermore, T3 does not include triaryl phosphine, triaryl arsine, triaryl stibine groups and the corresponding oxides.

Typically, the process of the invention affords compounds of formula (III) wherein T₁ is the same as T₃ in admixture with compounds of formula (III) wherein T₁ is different from T₃. Such mixtures can be submitted to known separation techniques, for instance to chemico-physical separations on porous supports like those disclosed in HILTON , RICHARD. Advanced Novel Separations. Business Communication Company Inc., 2000. LIBERTI , ARNALDO, et al. Chimica Analitica. Torino: UTET, 1980.. Encyclopedia of Chromatography. 3rd edition. CAZES, Jack, 2009. UGO, Renato. Analisi Chimica Strumentale. Guadagni, 1972. Mixtures of compounds of formula (III) may also be distilled or rectified.

As an alternative, such mixtures can be submitted to derivatization reactions as described in greater detail below to provide mixtures of derivatives which can be submitted to the aforementioned separation techniques.

In a first aspect, the expression "optionally substituted aromatic or heteroaromatic group" denotes an aromatic or heteroaromatic group optionally containing one or more substituents which do not undergo radical hydrogen removal. Compounds of formula (III) containing such substituted aromatic or heteroaromatic groups are obtained by reaction between the peroxide (P) and an optionally substituted aromatic or heteroaromatic compound as defined above. In a second aspect, the expression "optionally substituted aromatic or heteroaromatic group" denotes an aromatic or heteroaromatic group optionally containing one or more substituents which undergo radical hydrogen removal; such substituents can be formed or introduced by submitting a compound of formula (III) obtained by reaction between the peroxide (P) and an aromatic or heteroaromatic compound optionally containing one or more substituents which do not undergo radical hydrogen removal to derivatization reactions, as described below in greater detail.

Typically, in the compounds of formula (III), chain (R_{f}) has a number-average molecular weight selected from 500 to 10,000, preferably from 800 to 5,000.

In the compounds of formula (III), chain (R_{f}) is preferably selected from any one of the following:
chain (R_{fA}) having formula:

   -(CF₂CF(CF₃)O)ₐ(CFXO)_{b}- or -CF₂CF(CF₃)O)ₐ(CFXO)_{b}-CF₂(R"_{f})CF₂-O-(CF₂CF(CF₃)O)ₐ(CFXO)_{b}-,

   in which R"_{f} is a fluoroalkenyl group containing from 1 to 4 carbon atoms;
   X is as defined above; a and b are integers selected in such a way that the number-average molecular weight is within the range indicated above; a/b is between 10 and 100;
chain (R_{fB}) having formula:

   -(CF₂CF₂O)_{c}(CF₂O)_{d}(CF₂(CF₂)_{z}O)ₕ-

   in which c and d are integer and h is 0 or an integer, selected in such a way that the number-average molecular weight is within the range indicated above; c/d is between 0.1 and 10; h/(c+d) is between 0 and 0.05 and z is 2 or 3;
chain (R_{fC}) having formula:

   -(C₃F₆O)ₑ(CF₂CF₂O)_{f}(CFXO)_{g}-

   in which X is as defined above; e, f and g are integers selected in such a way that the number-average molecular weight is within the range indicated above; e/(f+g) is between 0.1 and 10; f/g is between 2 and 10;
chain (R_{fD}) having formula:

   -(CF₂(CF₂)_{z}O)ₛ-

   in which s is an integer selected in such a way that the number-average molecular weight is within the range indicated above and z has the meaning defined above;
chain (R_{fE}) having formula:

   -(CR₅R₆CF₂CF₂O)_{j'}- or -(CR₅R₆CF₂CF₂O)_{p'}-R"_{f}-O(CR₅R₆CF₂CF₂O)_{q'}-

   in which R₅ and R₆ are the same or different from one another and selected from H, Cl and perfluoroalkyl containing from 1 to 4 C atoms; R"_{f} is a fluoroalkenyl group containing from 1 to 4 C atoms; j', p' and q' are integers selected in such a way that the number-average molecular weight is within the range indicated above;
chain (R_{fF}) having formula:

   -(CF(CF₃)CF₂O)_{j"}-

   in which j" is an integer selected in such a way that the number average molecular weight is within the range indicated above.

More preferably, chain (R_{f}) is a chain of formula (R_{fA}) or (R_{fB}).

Most preferably, chain (R_{f}) is a chain (R_{fB}).

The compounds of formula (III) can be used as precursors for compounds of further functional derivatives. For instance, the compounds of formula (III) can be submitted to reactions whereby functional groups incompatible with the reaction between peroxide (P) and the aromatic or heteroaromatic compound are inserted on the aromatic or heteroaromatic group or whereby substituents present on the aromatic or heteroaromatic group are converted into further substituents. As an example, a nitro substituent on the aromatic group can be reduced to an amino group, which may in turn be converted into a further functional group (e.g. an alkylamino group or an amido group). Substituted aromatic groups can also be transformed into polyaromatic or polyheteroaromatic groups; for example, the compounds of formula (III) bearing 1,2-dinitrophenyl end groups can be submitted to a reduction reaction to provide compounds of formula (III) bearing 1,2-diaminophenyl end groups. Such compounds can be further converted into a compound of formula (III) bearing benzimidazole groups.

The compounds of formula (III) in which T₁ is different from T₃, in particular those in which T₁ is -C(O)F, can be submitted to derivatization reactions whereby the -C(O)F group is converted into a further functional group, e.g. a hydroxy group, a carboxylic group, an ester group, an aldehyde group or an amido group. These functional groups can in turn be converted into further functional or non-functional groups according to known methods. For the purposes of the present description, the term "functional group" is intended to denote a group of atoms determining the chemical behaviour of a family of compounds. Examples of functional compounds obtainable through derivatization reactions are those wherein T₁ is a group of formula:

-(A*)_{q*}-(T*)_{p*}

wherein:
- q* and p* are 0 or 1, with the proviso that at least one of them is other than 0;
- A* is a straight or branched alkyl chain, an (alkyl)clycloaliphatic or an (alkyl)aromatic chain when p* is 0 or a straight or branched alkylene chain, an (alkylene)clycloaliphatic or an (alkylene)aromatic chain when p* is 1;
- T* is a group consisting of or containing one or more groups selected from hydroxyl, ester, carboxy, thiol, alkylthio, amino, alkylamino, a silicon-containing group, cyano, isocyanate, alkenyl, vinyl, vinyl ether, vinylalkyl ether, aldehyde, keto, sulphonyl, sulphate, phosphonate, phosphate, carbonate, anhydride, halogen, oxime, hydrazine, guanidine, amide, acrylate, methacrylate, nitro and epoxide.

With regard to the definition of A*:
- a straight or branched alkyl or alkylene chain is preferably a straight or branched alkyl or alkylene chain containing from 1 to 20 carbon atoms;
- an (alkyl)cycloaliphatic or (alkylene)cycloaliphatic chain in which the alkyl or alkylene moiety is a straight or branched alkyl or alkylene moiety containing from 1 to 20 carbon atoms and the cycloaliphatic moiety has from 3 to 20 carbon atoms;
- an (alkyl)aromatic or (alkyene)aromatic chain in which the alkyl or alkylene moiety is as defined above and the aromatic moiety contains 5 to 20 carbon atoms.

The alkyl or alkylene chain optionally contains one or more -COO-, -COS- or -CONH- groups and/or one or more etheroatoms selected from N, P, S and O and in the cycloaliphatic or aromatic moiety one ore more carbon atoms is optionally replaced by etheroatoms selected from N, P, S and O.

Preferred groups A* in which the alkyl or alkylene chain contains one or more O atoms are (poly)alkyleneoxide chains containing repeating units of formula -CH₂CH₂O-, -CH₂CH(CH₃)O-, -(CH₂)₃O- or -(CH₂)₄O-, more preferably repeating units of formula -CH₂CH₂O-, -CH₂CH(CH₃)O-.

With regard to the definition of T*:
- a group containing more hydroxy groups is preferably a group of formula -CH(CH₂OH)₂;
- an ester group is preferably a group of formula -color'* in which R'* is a straight or branched alkyl chain containing from 1 to 20 carbon atoms and optionally containing fluorine atoms and/or a 3 to 20 member cycloaliphatic or heterocycloaliphatic moiety or a 5 to 20 member aromatic or heteroaromatic moiety;
- a group containing more ester groups is preferably a group of formula - CH(COOR'*)₂, in which R'* is as defined above;
- the term "carboxy" comprises also its alkali and alkali metal salts and acyl halides, preferably acyl chlorides;
- a group containing more carboxy groups is preferably a group of formula -CH(COOH)₂;
- an alkylthio group is preferably a group of formula -SR'*, in which R'* is as defined above;
- a silicon-containing group is preferably a group of formula -SiR'**_{d}*Q_{3-d} in which R'* is as defined above, *d* is 0 or an integer from 1 to 3, Q is an - O(CO)*_{d0}*R'* group and *d₀* is 0 or 1;
- a group containing more cyano groups is preferably a group or formula - CH(CN)₂;
- a vinyl alkyl ether group is preferably a group complying with formula - O(CH₂)_{m'*}CH=CH₂, in which m'* ranges from 1 to 18; more preferably, a vinyl ether group is a group complying with formula -OCH₂CH=CH_{2.} A group containing more vinyl ether groups is preferably a group of formula - CH[O(CH₂)_{m'*}CH=CH₂]₂, in which m'* is as defined above, more preferably a group of formula -CH[OCH₂CH=CH₂]₂;
- an alkylamino group comprises a monoalkylamino group preferably complying with formula -NHR'* and a dialkylamino group preferably complying with formula -NR'*₂ in which R'* is as defined above;
   a group containing more amino groups is preferably a group of formula - CH(CH₂NHR'*)₂ or a group of formula -CH(CH₂NR'*₂)₂ in which R'* is as defined above;
- the term aldehyde is meant to comprise also the corresponding acetal and thioacetal derivatives, such as dimethyl acetals and dimethylthioacetals;
- a keto group is preferably a group of formula -COR'* in which R'* is as defined above, including any ketal and thioketal derivatives, such as dimethyl acetals and dimethylthioacetals;
- a sulphonyl group is preferably a group of formula -SO₃H and any alkali or alkaline-hearth metal salts; -SO₂Hal, in which Hal is halogen as defined above, preferably chlorine; a group of formula -SO₂R'*, in which R'* is as defined above; a group of formula -SO₂R"*, in which R"* is a fluorinated straight or branched C₁-C₆alkyl chain, more preferably a group of formula - SO₂CF₃;
- a sulphate group is preferably a group of formula -OSO₃H and any alkali or alkaline-hearth metal salts, -OSO₂Hal or -OSO₂R'*, in which Hal and R'* are as defined above;
- a phosphonate group is preferably selected from -P(O)(OH)₂ and-P(O)(OH)(OR'*) and their respective alkali or alkaline-hearth metal salts; and -P(O)(OR'*)₂, in which R'* is as defined above;
- a phosphate group is preferably selected from -OP(O)(OH)₂;-OP(O)(OH)(OR'*) and their respective alkali or alkali-earth metal salts and
- OP(O)(OR'*)₂; in which R'* is as defined above.
- a carbonate group is preferably selected from -OC(O)OH and its alkali or alkali-earth metal salts, -OC(O)Hal in which Hal is as defined above and - OC(O)OR'*, in which R'* is as defined above;
- halogen is selected from fluorine, chlorine, bromine and iodine, iodine being particularly preferred;
- an anhydride group is preferably a group of formula -(O)C-O-C(O)-R'*, in which R'* is as defined above;
- an oxime group is preferably a group of formula -NH-OH or -NH-OR'*, in which R'* is as defined above;
- a hydrazino group is preferably a group of formula -NH-NH₂ or a group or formula -NH-NHR'*, in which R'* is as defined above;
- a guanidine group is preferably a group of formula -NH-C(=NH)-NH₂ or a group of formula -NH-C(=NH)-NHR'*, in which R'* is as defined above.

Examples of -(A*)_{q*}-(T*)_{p*} groups are those disclosed as -(A)_{q}-(T₂)ₚ groups in EP 2170968 A (SOLVAY SOLEXIS SPA).

The compounds of formula (III) and their derivatives are endowed with improved hydrolysis resistance at temperatures as high as 300°C, even in the presence of Lewis acids and metals; therefore, the compounds of formula (III) can be used as stabilizers for fluorinated oils and greases, preferably for (per)fluoropolyether oils and greases to be used as such temperatures and under harsh conditions. Furthermore, the compounds of formula (III) wherein one or both chain ends bear an aromatic group containing an -OCH₂O-, a -COOH, an -SH or an -NO₂ group are able to confer anti-wear properties to (per)fluoropolyether oils and greases.

Thus, the present description relates to a lubricant composition which comprises a compound of formula (III) as defined above in admixture with a (per)fluoropolyether oil.

Typically, the lubricant composition according to the description comprises:
(a) from 0.05 to 20% wt, preferably from 0.1 to 5% wt, more preferably from 0.5 to 2% wt of a compound of formula (III) as defined above or of a derivative thereof;
(b) a (per)fluoropolyether oil and, optionally
(c) one or more additives.

The above percentages are expressed with respect to the weight of the composition.

Preferably, the (per)fluoropolyether oil has a viscosity at 20°C between 10 and 4,000 cSt, more preferably from 30 to 2,000 cSt.

(Per)fluoropolyether oils for the preparation of the compositions herein described preferably comply with formulae (1a) - (8a) below:

(1a) E-O-(CF₂CF(CF₃)O)_{m'}(CFXO)_{n'}-E'

wherein
X is F or CF₃;
E and E', equal to or different from each other, are selected from -CF₃, - C₂F₅ or -C₃F₇; in one or both groups E and E' one fluorine atom can replaced with Cl and/or H;
m' and n' are integers such that the m'/n' ratio is between 20 and 1,000, n' being different from zero, selected in such as way that the product viscosity is as defined above; and wherein the -(CF₂CF(CF₃)O)- and (CFXO) units are statistically distributed along the chain.

The (per)fluoropolyethers of formula (1a) can be obtained by perfluoropropene photooxidation, as described in GB 1104432 (LESLIE THOMAS COOPER JOHNSON), and by subsequent conversion of the end groups, as disclosed in GB 1226566 (MONTECATINI EDISON SPA)

(2a) C₃F₇O(CF(CF₃)CF₂O)_{o'}-D

wherein:
D is -C₂F₅ or -C₃F₇, wherein one fluorine atom can be replaced by Cl or H;
o' is an integer selected in such a way that the viscosity is in the above-defined range.

The (per)fluoropolyethers of formula (2a) can be prepared by ionic oligomerization of the perfluoropropylenoxide and subsequent treatment with fluorine, as described in US 3242218 (DU PONT);

(3a) {C₃F₇O-(CF(CF₃)CF₂O)_{p'}-CF(CF₃)-}₂

wherein:
p' is an integer such that the product viscosity is in the above-defined range and wherein one F atom of one or both the C₃F₇ end groups can be replaced with Cl and/or H.

The (per)fluoropolyethers of formula (3a) can be obtained by ionic telomerization of the perfluoropropylenoxide and subsequent photochemical dimerization, as reported in US 3214478 (DU PONT) ;

(4a) E-O-(CF₂CF(CF₃)O)_{q'}(C₂F₄O)_{r'}(CFXO)_{s'}-E'

wherein:
X is F or CF₃;
E and E', equal to or different from each other, are as defined above;
q', r' and s' are 0 or integers, selected in such a way that the product viscosity is as defined above.

The (per)fluoropolyethers of formula (4a) are obtainable by photooxidation of a mixture of C₃F₆ and C₂F₄ and subsequent treatment with fluorine, as described in US 3715378 (MONTEDISON SPA) ;

(5a) E-O-(C₂F₄O)_{t'}(CF₂O)_{u'}-E'

wherein:
E and E', equal to or different from each other, are as defined above;
t' and u' are integers such that the t'/u' ratio is between 0.1 and 5 and the product viscosity is in the above-defined range.

The (per)fluoropolyethers of formula (5a) can be obtained by C₂F₄ photooxidation, as reported in US 3715378 (MONTEDISON SPA) and subsequent treatment with fluorine, as described in US 3665041 (MONTEDISON SPA) ;

(6a) E-O-(CF₂CF₂CF₂O)_{v'}-E'

wherein:
E and E', equal to or different from each other, are as defined above;
v' is an integer selected in such a way that the that the product viscosity is in the above-defined range.

The (per)fluoropolyethers of formula (6a) can be obtained as described in EP 148482 A (DAIKIN IND LTD) ;

(7a) D-O-(CF₂CF₂O)_{z'}-D'

wherein:
D and D', equal to or different from each other, are selected from C₂F₅ or C₃F₇; in D and/or D' one fluorine atom be replaced by Cl or H;
z' is an integer selected in such a way that the product viscosity is as defined above.

The (per)fluoropolyethers of formula (7a) can be obtained as described in US 4523039 (UNIV TEXAS) USP 4,523,039;

(8a) E₁-O(CF₂O)ₙ(CF₂CF₂O)ₘ(CF₂CF₂CF₂O)ₚ(CF₂CF₂CF₂CF₂O)_{q}-E₂

wherein:
E₁ and E₂ are perfluoroalkyl end groups equal to or different from one another, having formula -(CF₂)_{z}CF₃ wherein z is an integer from 0 to 3;
n, m, p, q are integers equal to or different from each other between 0 and 100, selected in such a way that the oil viscosity is as defined above and that: the m/n ratio is between 2 and 20; the (p+q)/(n+m+p+q) ratio is between 0.05 and 0.2; the n/(n+m+p+q) ratio is between 0.05 and 0.40, with the proviso that (n+m+p+q) is different from 0.

The (per)fluoropolyether of formula (8a) can be obtained as described in EP 1454938 A (SOLVAY SOLEXIS SPA).

Preferably, the (per)fluoropolyether oil is selected from those of formulae (1a), (4a), (5a), (8a) and mixtures thereof; more preferably, the (per)fluoropolyether oil is selected from the compounds of formulae (5a) and (8a).

The additives optionally present in the lubricant compositions herein described are selected from those commonly used in fluorinated lubricant compositions. The lubricant compositions according to the present description can optionally contain further additives, for example selected from antirust agents, antioxidants, thermal stabilizers, pour point depressants, antiwear agents, including those for high pressures, dispersants, tracers, dyestuffs, talc and inorganic fillers. Examples of dispersants are, for example, surfactants, preferably non-ionic surfactants, more preferably (per)fluoropolyether surfactants and (per)fluoroalkyl surfactants. Examples anti-wear additives are phosphazene derivatives of (per)fluoropolyethers, like those disclosed in EP 1336614 A (SOLVAY SOLEXIS SPA).

The lubricant compositions according to the present description can be in the form of oils or greases. Those in the form of greases contain a thickening agent, for example PTFE, talc, silica, boron nitride, polyurea, alkali or alkali-earth metals terephthalate, calcium and lithium soaps and complexes thereof; among them, PTFE is preferred.

Exemplary and non limiting embodiments of the invention are illustrated in detail in the following experimental section.

Should the disclosure of any patents, patent applications, and publications which are incorporated herein by reference conflict with the description of the present application to the extent that it may render a term unclear, the present description shall take precedence.

### EXPERIMENTAL SECTION

### Materials and methods

Benzene, bromobenzene, fluorobenzene, bis-trifluormethylbenzene, 1,2-dinitrobenzene, pyrocathecol-*bis*(trifluoromethyl)acetate, benzoic acid and the solvents were purchased from Sigma Aldrich^{®} and used as received. Peroxidic PFPEs were synthesised according to US 3715378 **.**

### 1. Determination of the peroxide content

The peroxide content was determined by uodometric titration according to the following method. A definite amount of sample (between 1 to 10 grams) was dissolved in about 20 ml Galden^{®} ZT 130 PFPE and added with 1 ml glacial acetic acid and 30 ml of a Nal solution (5%w/w) in isopropanol. The resulting suspension was left under stirring for 15 minutes and the iodine formed in the reaction was titrated with an aqueous solution of Na₂S₂O₃ having known concentration, using a Mettler^{®} DL40 instrument for potentiometric titration, equipped with a platinum electrode and with a reference electrode. The sensitivity limit of the method is 0.0002.

### 2. Stability test of PFPE oils to Lewis acids

The stability of PFPE oils to Lewis acids was evaluated according to the following procedure. 5 g oil, optionally containing a compound according to the present invention at the concentration indicated in the examples, and 0.1 g AlF₃ were placed in a 10 ml glass tube, which was subsequently weighed and closed with a holed cap allowing the insertion of a small PTFE tube for conveying any decomposition products to a quenching 0.1 NaOH solution placed into a suitable glass flask. The test tube was then warmed up to 250°C for 24 hrs; after this time, it was cooled and weighed. The test result is expressed as weight loss percentage over the starting oil.

### 3. Microoxidation test

The microoxidation test referred to in the examples was carried out with the equipment disclosed in: SNYDER, Karl, et al. Development of Polyperfluoroalkylethers as High Temperature Lubricants and Hydraulic Fluids. ALSLE Transactions. 1975, vol.13, no.3, p.171-180. under the following conditions:
- PFPE oil: Fomblin^{®} M30 PFPE, having a kinematic viscosity at 20°C of 270 cST (2.7 x 10⁸ m²/s) and an acidity, determined as explained below, lower than 0.01 mg KOH/g;
- test temperature: 300°C (if not otherwise indicated);
- testing time: 24 hrs
- air flux: 1 l/hr
- metals immersed in the oil: stainless steel (AISI 304) and a titanium alloy (6A14V).

The test glass tube containing a weighed amount of metal was filled up with the oil to be tested and, optionally, a compound according to the present invention, then it was weighed and heated up to the test temperature. After a determined time the tube was cooled down to room temperature and weighed again. The loss weight percentage was determined calculating the difference before and after the thermal treatment. The oil was then recovered to measure acidity and kinematic viscosity. The surface aspect of the metal was also visually evaluated.

### 4. Thermal oxidation test

Thermal stability was evaluated by placing a known aliquot (between 1 and 10 g) of a compound according to the present invention into a glass autoclave and the temperature was maintained at 300°C (unless otherwise indicated) for 24 hours.

At the end of the test, the autoclave was cooled and optionally depressurized by venting off low-boiling by-products.

Loss weight is expressed as percentage difference between the amount of the compound according to the invention between before and after the test.

After the test, the compound was also analysed by ¹⁹F and ¹H NMR.

### 5. Test of hydrolytic stability

Hydrolytic stability was evaluated by placing a known aliquot (between 1 and 10 g) of a compound according to the present invention (about 10 g) or of oil/grease containing 1 % wt additive of a compound according to the present invention and 1 g of water, in a glass autoclave, maintaining the temperature at 80°C (unless otherwise indicated) for 240 hours.

At the end of the test, the autoclave was cooled and optionally depressurized by venting off low-boiling by-products.

The test residue was analysed by ¹⁹F and ¹H NMR and the hydrolysis percentage was determined as disclosed in TONELLI, Claudio, et al. Perfluoropolyether alkyl diesters: Structure effects of the alkyl group on the kinetics of the hydrolysis reactions. Journal of Polymer Science, Polymer Chemistry, PartA. 2002, vol.40, no.23, p.4266-4280.

### PREPARATIVE EXAMPLES

### Example 1 - Reaction between bromobenzene and a PFPE peroxide of formula (IIA)

3 g bromobenzene and 9.4 g (corresponding to 6.3 mmol peroxide groups) PFPE peroxide of formula:

CF₃O(C₂F₄O)ᵣ(CF₂O)ₛ(O)₂₇COF r/s = 1

with Mn = 40,000 and containing an average number of peroxide groups per chain of 27 (corresponding to PO = 0.7)
were loaded into a glass autoclave equipped with manometer and magnetic stirrer under nitrogen atmosphere.

The temperature was raised to 200°C and the mixture was left under stirring for 8 hrs; an increase of internal pressure (a few atmospheres) was observed. The reaction mixture was cooled down to room temperature to recover 11 g of product with no peroxide groups (¹⁹F and ¹H NMR analysis). ¹⁹F NMR analysis confirmed the presence of a -CF₂- group directly linked to the aromatic ring (signal at -66/-68 ppm); a comparison between the ¹⁹F-NMR and ¹H-NMR analysis confirmed that -CF₂- group was the only fluorinated substituent on the aromatic ring. No signals typical of the -CF₂-O-Ar sequence were detected.

Upon completion of the reaction, the recovered mixture contained, in addition to unreacted bromobenzene, the following products:
- 20% mol FOC(C₂F₄)_{c'} (CF₂O)_{d'}COF (a);
- 40% mol Br-benzeneCF₂O(C₂F₄)_{c'}(CF₂O)_{d'}COF (b);
- 40% mol Br-benzeneCF₂O(C₂F₄)_{c'}(CF₂O)_{d'}CF₂-benzene-Br (c) in which c'/d' = 0.95 and the (C₂F₄)_{c'}(CF₂O)_{d'} chain had an average numerical molecular weight of 1.550.

It stems from the above that the conversion with respect to the peroxide groups was 100% and the selectivity with respect to products (b) and (c) was 80%.

### Example 2 - Reaction between fluorobenzene and a PFPE peroxide of formula (IIa)

The same procedure as in example 1 was followed, using 1.8 g fluorobenzene instead of bromobenzene.

Upon completion of the reaction, the recovered mixture (10.1 g) contained, in addition to unreacted fluorobenzene, the following products:
- 25% mol FOC(C₂F₄)_{c'}(CF₂O)_{d'}COF (a);
- 50% mol F-benzeneCF₂O(C₂F₄)_{c'}(CF₂O)_{d'}COF (d);
- 25% mol F-benzeneCF₂O(C₂F₄)_{c'}(CF₂O)_{d'}CF_{2'}benzene-F (e) in which c'/d' = 0.95 and the (C₂F₄)_{c'}(CF₂O)_{d'} chain had an average numerical molecular weight of 1.550.

No other products in the mixture were detected with ¹⁹F and ¹H NMR analysis.

It stems from the above that the conversion with respect to the peroxide groups was 100% and the selectivity with respect to products (d) + (e) was 75%.

### Example 3 - Separation of the components of the mixture from example 2

The reaction mixture from example 2 was rectified with a 20 theoretical plates column. By progressively applying vacuum until a residual pressure of 10⁻³ kPa and keeping the heater temperature at 250°C, a fraction containing about 2 g fluorobenzene, product (a) and 5% by weight of compound (d) was recovered in a temperature range between 100 and 200°C.

The rectification residue (about 9 g), consisting of a mixture of products (d) (50%) and (e) (50%), was submitted to hydrolysis with 100 ml water at 50° C for 8 hrs; thereafter, the upper aqueous phase was separated from the lower organic phase and washed with two 30 ml aliquots of H-Galden^{®} PFPE. The organic phases were pooled and analyzed by IR spectroscopy: complete conversion of the -C(O)F terminal groups into -COOH groups was observed.

The hydrolysed solution was loaded into a column containing 20 g silica gel (70-230 mesh, 60 A) and eluted with 200 ml H-Galden^{®} PFPE. The eluate contained only compound (e), which was isolated (4 g) after solvent evaporation. The stationary phase containing compound (d) was recovered with 500 ml of an HF aqueous solution left under stirring for 8 hrs; silica gels dissolves as hexafluorosilicic acid. By three subsequent extractions with 200 ml (in total) H-Galden^{®} PFPE, 4 g product (d) were isolated

### Example 4 - Conversion of compound (e) obtained from examples 2 and 3 above into the corresponding diethylamino derivative.

3 g compound (e), obtained following examples 2 and 3 above, was dissolved in 10 ml H-Galden^{®} PFPE and added with a 100% excess triethylamine and a catalytic amount of potassium tert-butylate (5% mol with respect to the aromatic groups).

The resulting mixture is allowed to react for two hrs at room temperature, then the temperature is raised to 55°C (triethylamine boiling point) and the reaction is continued for two further hrs. After this time the mixture is cooled down to room temperature, filtered and washed with 10 ml aqueous HCl (2% solution). The organic phase was separated, dried with 2 g Na₂SO₄, filtered and the solvent was removed by distillation to afford 2.8 g of product (f) having formula:

(C₂H₅)₂N-benzeneCF₂O(C₂F₄)_{c'}(CF₂O)_{d'}CF₂-benzene-F-N(C₂H₅)₂ (f)

in which c'/d' = 0.95 and the (C₂F₄)_{c'}(CF₂O)_{d'} chain had an average numerical molecular weight of 1.550 (structure determined through ¹⁹F and ¹H NMR analysis).

### Example 5 - Reaction between bis-trifluoromethylbenzene and a PFPE peroxide of formula (IIa)

The procedure illustrated in example 1 was followed, using 5 g (23 mmol) *bis*-trifluoromethylbenzene and 11 g of the same peroxide of formula CF₃O(C₂F₄O)_{c}(CF₂O)_{d}(O)₂₇COF (7.4 mmol peroxide groups).

Upon completion of the reaction, the recovered mixture (14 g) was analysed by ¹⁹F and ¹H NMR. The results confirmed the absence of peroxide groups and that the mixture contained, in addition to unreacted *bis-*trifluoromethylbenzene, the following products:
- 19% mol FOC(C₂F₄)_{c'}(CF₂O)_{d'}COF (a);
- 41 % mol (CF₃)₂-benzeneCF₂O(C₂F₄)_{c'}(CF₂O)_{d'}COF (g);
- 40% mol (CF₃)₂-benzeneCF₂O(C₂F₄)_{c'}(CF₂O)_{d'}-benzene-(CF₃)₂ (h);
   in which c'/d' = 0.95 and the (C₂F₄)_{c'}(CF₂O)_{d'} chain had an average numerical molecular weight of 1.550.

No other compound was detected.

It stems from the above that the conversion with respect to the peroxide groups was 100% and the selectivity with respect to products (g) + (h) was 81%.

### Example 6 - Separation of the components of the mixture of example 5

Following the procedure illustrated in example 3 above, *bis-*trifluoromethylbenzene and product (a) (2.4 g) were obtained as first distillation fraction. The remaining mixture of products (g) and (h) was hydrolysed and submitted to column chromatography on silica gel to separate compound (g) (5.5 g). Compound (f) (5.3 g) was recovered from the silica gel in the same way as disclosed in example 3.

### Example 7 - Reaction between benzene and a PFPE peroxide of formula (IIa)

The procedure illustrated in example 1 was followed, using 2 g benzene (26 mmol) and 11 g of the same peroxide of formula CF₃O(C₂F₄O)_{c}(CF₂O)_{d}(O)₂₇COF (7.4 mmol peroxide groups).

Upon completion of the reaction, the recovered mixture (12 g) was analysed by ¹⁹F and ¹H NMR. The results confirmed the absence of peroxide groups and that the mixture contained, in addition to unreacted benzene, the following products:
- 29% mol FOC(C₂F₄)_{c'}(CF₂O)_{d'}COF (a);
- 36% mol benzeneCF₂O(C₂F₄)_{c'}(CF₂O)_{d'}COF (i);
- 35% mol benzeneCF₂O(C₂F₄)_{c'}(CF₂O)_{d'}benzene (l);
   in which c'/d' = 0.95 and the (C₂F₄)_{c'}(CF₂O)_{d'} chain had an average numerical molecular weight of 1.550.

No other products were detected in the mixture.

It stems from the above that the conversion with respect to the peroxide groups was 100% and the selectivity with respect to products (i) + (l) was 71%.

The above product mixture was rectified to remove the unreacted benzene, then hydrolysed, diluted to 10% wt in H-Galden^{®} PFPE and chromatographed on silica gel as illustrated in example 3. After evaporation of the solvent, 4 g product (l) was isolated and submitted to nitration according to a known procedure, to afford compound (m), having formula:

NO₂-benzene-CF₂O(C₂F₄)_{c'}(CF₂O)_{d'}-benzene-NO₂;

in which c'/d' = 0.95 and the (C₂F₄)_{c'}(CF₂O)_{d'} chain had an average numerical molecular weight of 1.550.

### Example 8 - Reaction between 1,2-dinitrobenzene and a peroxide of formula (II-A)

The procedure illustrated in example 1 was followed, using a 50 ml glass autoclave, 4 g 1,2-dinitrobenzene (24 mmol) and 11 g of the same peroxide of formula CF₃O(C₂F₄O)_{c}(CF₂O)_{d}(O)₂₇COF (7.4 mmol peroxide groups).

Upon completion of the reaction, the recovered mixture (about 12.8 g) was analysed by ¹⁹F and ¹H NMR. The results confirmed the absence of peroxide groups and that the mixture contained, in addition to unreacted dinitrobenzene, the following products:
- 28% mol FOC(C₂F₄)_{c'}(CF₂O)_{d'}COF (a);
- 36% mol (NO₂)₂-benzeneCF₂O(C₂F₄)_{c'}(CF₂O)_{d'}COF (n)
- 36% (NO₂)₂-benzene-CF₂O(C₂F₄)_{c'}(CF₂O)_{d'}-benzene-(NO₂)₂ (o) in which c'/d' = 0.95 and the (C₂F₄)_{c'}(CF₂O)_{d'} chain had an average numerical molecular weight of 1.550.

No other products were detected in the mixture.

It stems from the above that the conversion with respect to the peroxide groups was 100% and the selectivity with respect to products (n) + (o) was 72%.

The above product mixture was rectified, hydrolysed and submitted to column chromatography according to example 3; the eluate was was collected and the solvent was evaporated off, to recover 4.1 g compound (o), which was treated with Fe/HCl according to a known procedure to provide the corresponding diamino derivative. This compound was further reacted with HOT (CONTROLLARE) formic acid and submitted to a cyclization reaction to provide the corresponding benzimidazole derivative of formula (p):

benzimidazole-CF₂O(C₂F₄)_{c'}(CF₂O)_{d'}-benzimidazole (p)

The overall yield amounted to 80% wt with respect to the starting peroxide.

### Example 9 (Comparative example) - Synthesis of a PFPE derivative having benzimidazole groups bound to the PFPE chain via an -CH₂O-bridge

20 g (26 meq) Fomblin^{®} Z DOL PFPE of formula:

HOCH₂CF₂O(C₂F₄O)_{c}(CF₂O)_{d}CF₂CH₂OH,

with c/d = 1 and average molecular weight of 1,500 was charged into a 100 ml reactor equipped with magnetic stirrer, thermometer and condenser and added with a 10% wt solution of potassium *tert*-butylate (30 meq) in *tert*-butanol. The resulting mixture was heated to 40°C and left under stirring for 1 hr. After this time, 7 g 1-chloro-3,4-dinitrobenzene was added and the reaction was continued at 50°C for 3 hrs. The reaction mass was neutralized and repeatedly washed with water, then the organic phase was separated and dried, to afford 24 g of a product of formula (q)

(NO₂)₂-benzene-CH₂CF₂O(C₂F₄O)_{c'}(CF₂O)_{d'}CF₂CH₂-benzene-(NO₂)₂ (q)

with c/d = 0.95 and the -(C₂F₄O)_{c'}(CF₂O)_{d'}- had a number average molecular weight of 1,550.

This compound was converted into the corresponding benzimidazole derivative of formula (r)

benzimidazole-CH₂CF₂O(C₂F₄O)_{c'}(CF₂O)_{d'}CF₂CH₂-benzimidazole (r)

following the same reaction sequence as indicated in example 8 above.

The overall yield amounted to 80% wt with respect to starting Fomblin^{®} Z DOL PFPE.

### Example 10 - Reaction between pyrocathecol-bis(trifluoromethyl)acetate and a PFPE peroxide of formula (II-A)

The procedure illustrated in example 1 was followed, using 4.8 g (16 mmol) pyrocathecol-bis(trifluoromethyl)acetate (PLEASE CHECK NOMENCLATURE) and 9.0 g (6.1 mmol peroxide groups) peroxide of formula of the same peroxide of formula CF₃O(C₂F₄O)_{c}(CF₂O)_{d}(O)₂₇COF.

Upon completion of the reaction, the recovered mixture (about 10.2 g) was analysed by ¹⁹F and ¹H NMR. The results confirmed the absence of peroxide groups and that the mixture contained, in addition to unreacted pyrocathecol-bis(trifluoromethyl)acetate, the following products:
- 18% mol FOC(C₂F₄)_{c'}(CF₂O)_{d'}COF (a);
- 40% mol pyrocathecol-bis(trifluoromethyl)acetate-CF₂O(C₂F₄)_{c'}(CF₂O)_{d'} COF (s);
- 42% mol pyrocathecol-*bis*(trifluoromethyl)acetate-CF₂O(C₂F₄)_{c'}(CF₂O)_{d'} CF₂-pyrocathecol-*bis*(trifluoromethyl)acetate (t)
   in which c'/d' = 0.95 and the (C₂F₄)_{c'}(CF₂O)_{d'} chain had an average numerical molecular weight of 1.550.

No other products were detected in the mixture.

It stems from the above that the conversion with respect to the peroxide groups was 100% and the selectivity with respect to products (s) and (t) was 82%.

The mixture was then rectified, hydrolysed and submitted to column chromatography as disclosed in example 3, to afford 3.5 g compound (t).

### Example 11 - Hydrolysis of compound (t) obtained from example 10

3.5 g compound (t) obtained according to example 10 and 10 g of a 10% wt solution in NaOH were loaded into a 20 ml reactor equipped with thermometer and magnetic stirrer under nitrogen atmosphere. The temperature was increased to 50°C and the mixture was left under stirring for 4 hrs. After this time the mixture was cooled down to room temperature, neutralized and repeatedly washed with water. The lower organic phase was separated, dried with Na₂SO₄ and any solvent residues were removed by distillation, to afford 3 g of a compound of formula (u):

pyrocathecol-CF₂O(C₂F₄)_{c'}(CF₂O)_{d'}CF₂-pyrocathecol (u)

in which c'/d' = 0.95 and the (C₂F₄)_{c'}(CF₂O)_{d'} chain had an average numerical molecular weight of 1.550.

Compound (u) was treated with methylene chloride and NaOH in DMSO according to a known procedure to obtain compound (v) of formula:

benzodioxole-CF₂O(C₂F₄)_{c'}(CF₂O)_{d'}CF₂-benzodioxole

in which c'/d' = 0.95 and the (C₂F₄)_{c'}(CF₂O)_{d'} chain had an average numerical molecular weight of 1.550.

### Example 12 - Reaction between benzoic acid and a PFPE peroxide of formula (II-A)

### Method 1

A mixture of benzoic acid (2 g, 16 mmol) and CF₃(C₂F₄O)_{c}(CF₂)_{d}(O)₂₂-COF (6.7 g, 6 mmol of peroxide groups, c/d = 0.96; Mn = 24,000; PO = 1.46) was prepared in a 20 ml glass flask equipped with magnetic stirrer and joint to a nitrogen feeding line.

Nitrogen was fed into the flask for 30 minutes (5Nl/h) under magnetic stirring. Then, keeping up the nitrogen feeding, the mixture was immersed in oil bath at 205°C (temperature in the flask = 200°C) and kept under these conditions for 135 minutes. After this time the reaction mixture was cooled down to room temperature, then a sample was taken, dissolved in freon A113 and analyzed by ¹H and ¹⁹F NMR. The NMR analysis indicated that the peroxidic groups were completely converted (P.O.=0) and that, in addition to excess benzoic acid, the mixture contained:
- 43 % mol FOC(C₂F₄O)_{c'}(CF₂O)_{d'}-COF (v);
- 45 % mol HOOC-Ph-CF₂O-(C₂F₄O)_{c'}(CF₂O)_{d'} -COF (z);
- 12 % mol HOOC-Ph-CF₂O-(C₂F₄O)_{c'}(CF₂O)_{d'} -CF₂-Ph-COOH
   wherein c'/d'=0.95 and -(C₂F₄O)_{c'}(CF₂O)_{d'} - had an average Mₙ=1.550. The selectivity of products (v) + (z) was of about 57 %.

No products containing ether bonds between the perfluopolyetheric chain and the aromatic ring were detected in the mixture.

### Method 2

The same procedure as for method 1 was followed, with the difference that 2.7 g (8 mmol) decafluorobiphenyl was included in the starting mixture of benzoic acid and peroxide.

NMR analysis of a sample taken from the reaction mixture and dissolved in freon A113 indicated that the peroxidic groups were completely converted (PO=0) and that, in addition to excess benzoic acid, the mixture contained:
- 33% mol FOC(C₂F₄O)_{c'}(CF₂O)_{d'}-COF (v);
- 49% mol HOOC-Ph-CF₂O-(C₂F₄O)_{c'}(CF₂O)_{d'} -COF (z);
- 18% mol HOOC-Ph-CF₂O-(C₂F₄O)_{c'}CF₂O)_{d'} -CF₂-Ph-COOH
   wherein c'/d'=0.96 and -(C₂F₄O)_{c'}(CF₂O)_{d'} - had an average Mₙ=1440.

The selectivity of product (v) + (z) was of about 67%.

Neither products containing ether bonds between the perfluopolyetheric chain and the aromatic ring nor reaction product with decafluorobiphenyl were detected in the mixture.

### Method 3

A mixture of benzoic acid (10 g, 80 mmol) and decafluorobiphenyl (13.5 g, 40 mmol) was prepared in a 100 ml glass flask equipped with magnetic stirrer, a 25 ml dropping funnel and the gas feeding line. The was filled with CF₃(C₂F₄O)_{c}(CF₂)_{d}(O)₂₂-COF (33.5 g, 30 mmol of peroxide groups)

Nitrogen was fed into the flask for 30 minutes at 5Nl/h under magnetic stirring. After this time, nitrogen flux was reduced to 2 Nl/h and the mixture was heated to 220°C, then the peroxide was added drop-by-drop at constant rate over 2 hours, keeping the temperature at 220°C. This temperature was maintained for 1 further hour after the end of the addition, then the reaction mixture was cooled down to room temperature. A sample was taken, dissolved in freon A113 and analyzed by ¹H and ¹⁹F NMR. NMR analysis indicated that the peroxidic groups were completely converted (PO=0) and that the product mixture, further to an excess of benzoic acid, contained:
- 40% mol FOC(C₂F₄O)_{c'}(CF₂O)_{d'}-COF
- 46% mol HOOC-Ph-CF₂O-(C₂F₄O)_{c'}(CF₂O)_{d'}-COF
- 14% molar HOOC-Ph-CF₂O-(C₂F₄O)_{c'}(CF₂O)_{d'}-CF₂-Ph-COOH
   wherein c'/d'=0.96 and -(C₂F₄O)_{c'}(CF₂O)_{d'} - had an average Mₙ=1,400.

The selectivity of products (v) + (z) was of about 60 %.

Neither products with ether bonds between the perfluopolyetheric chain and the aromatic ring nor reaction products with decafluorobiphenyl were detected in the mixture.

## Claims

1. A process for the manufacture of a (per)fluoropolyether compounds having two chain ends, wherein one or both chain ends is a -CF₂- group covalently bound to an optionally substituted aromatic or heteroaromatic group through a -carbon-carbon- bond, said process comprising the reaction of a (per)fluoropolyether peroxide [peroxide (P)] with an optionally substituted aromatic or heteroaromatic compound, wherein
said aromatic compound is benzene, biphenyl, phenantrene, anthracene, benzoic acid, fluorobenzene, bromobenzene, trifluoromethylbenzene, nitrobenzene, methyl- or ethyl-benzoate, dimethylterephthalate, and pirocathecol;
said aromatic or heteroaromatic compound can bear one or more substituents, which may be the same or different from one another, said substituents on the aromatic compound being halogen, (per)haloalkyl, nitro, amino, amido, nitrile, ester groups and phosphorous- and sulphur-containing groups.

2. The process according to claim 1 wherein the (per)fluoropolyether peroxide [peroxide (P)] comprises at least one peroxidic moiety comprised between sp³ carbon atoms and at least one fully or partially fluorinated polyoxyalkene chain (chain R_{f}), i.e. a fluorocarbon segment comprising ether linkages in the main chain.

3. The process according to claim 2 wherein chain R_{f} comprises repeating units R°, statistically distributed along the chain, said repeating units being chosen among the group consisting of:
(i) (CFXO), wherein X is F or CF₃;
(ii) (CF₂CF₂O);
(iii) (C₃F₆O) units of formula (CF₂CF₂CF₂O), (CF₂CF(CF₃)O) and (CF(CF₃)CF₂O);
(iv) (CF₂CF₂CF₂CF₂O);
(v) (CR₅R₆CF₂CF₂O), wherein R₅ and R₆, which may be identical or different, are chosen from H, Cl and perfluoroalkyl containing from 1 to 4 carbon atoms;
(vi) -(CF₂)ⱼ-CFZ-O- wherein j is an integer from 0 to 3 and Z is a group of general formula -OR_{f}'T, wherein R_{f}' is a fluoropolyoxyalkene chain comprising a number of repeating units from 0 to 10, said recurring units being chosen among the followings : -CFXO- , -CF₂CFXO-, -CF₂CF₂CF₂O-, - CF₂CF₂CF₂CF₂O-, wherein X is independently F or CF₃ and T is a C₁-C₃ perfluoroalkyl group.

4. The process according to claim 3 in which peroxide (P) complies with formula (I) here below:
A-O-(CFXO)_{c1}(CF₂CF₂O)_{c2}(C₃F₆O)_{c3}(CF₂CF₂CF₂CF₂O)_{c4}(O)ₚ-B formula (I)
wherein:
- A and B, equal to or different from each other, are independently selected from -CF₃, -CF₂-CF₃, -CF₂Cl, -CF₂CF₂Cl, -CF(CF₃)COF, -CF₂-COF, -COF;
- X is F or CF₃;
- c1, c2 , c3, and c4, equal or different from each other, are independently integers ≥0 and p is an integer > 0, wherein c1, c2 , c3, c4 and p are selected in such a way that the average number molecular weight of the peroxide ranges from 300 to 150,000 and p is selected in such as way that the content of peroxide groups -O-O,- or oxidizing power (OP) is between 0.02 % and 4 %.

5. The process according to any one of the preceding claims which is carried out under continuous, semi-continuous or discontinuous conditions.

6. The process according to any one of the preceding claims which is carried out at a temperature ranging from -80°C to 300°C.

7. The process according to claim 6 which is carried out at a temperature ranging from -80°C to 150°C and under UV catalysis.

8. The process according to any one of the preceding claims which is carried out in bulk or in the presence of a solvent.

## Patentansprüche

1. Verfahren zur Herstellung einer (Per)fluorpolyetherverbindung mit zwei Kettenenden, wobei es sich bei einem Kettenende oder beiden Kettenenden um eine über eine Kohlenstoff-Kohlenstoff-Bindung kovalent an eine gegebenenfalls substituierte aromatische oder heteroaromatische Gruppe gebundene -CF₂-Crruppe handelt,
bei dem man ein (Per)fluorpolyetherperoxid [Peroxid (P)] mit einer gegebenenfalls substituierten aromatischen oder heteroaromatischen Verbindung umsetzt, wobei
es sich bei der aromatischen Verbindung um Benzol, Biphenyl, Phenanthren, Anthracen, Benzoesäure, Fluorbenzol, Brombenzol, Trifluormethylbenzol, Nitrobenzol, Methyl- oder Ethylbenzoat, Dimethylterephthalat und Pyrocatechol handelt;
die aromatische oder heteroaromatische Verbindung einen oder mehrere Substituenten, die gleich oder voneinander verschieden sind, tragen kann, wobei es sich bei den Substituenten an der aromatischen Verbindung um Halogen-, (Per)halogenalkyl-, Nitro-, Amino-, Amido-, Nitril-, Estergruppen und phosphor- und schwefelhaltige Gruppen handelt.

2. Verfahren nach Anspruch 1, bei dem das (Per)fluorpolyetherperoxid [Peroxid (P)] mindestens eine peroxidische Gruppierung zwischen sp³-Kohlenstoffatomen und mindestens eine voll- oder teilfluorierte Polyoxyalkylenkette (Kette R_{f}), d.h. ein Fluorkohlenstoffsegment mit Etherbindungen in der Hauptkette, umfasst.

3. Verfahren nach Anspruch 2, bei dem die Kette R_{f} statistisch entlang der Kette verteilte Wiederholungseinheiten R° umfasst, wobei die Wiederholungseinheiten aus der Gruppe bestehend aus
(i) (CFXO), wobei X für F oder CF₃ steht;
(ii) (CF₂CF₂O),
(iii) (C₃F₆O)-Einheiten der Formel (CF₂CF₂CF₂O), (CF₂CF(CF₃)O) und (CF(CF₃)CF₂O);
(iv) (CF₂CF₂CF₂CF₂O),
(v) (CR₅R₆CF₂CF₂O), wobei R₅ und R₆, die gleich oder voneinander verschieden sein können, aus H, Cl und Perifluoralkyl mit 1 bis 4 Kohlenstoffatomen ausgewählt sind;
(vi) -(CF₂)ⱼ-CFZ-O-, worin j für eine ganze Zahl von 0 bis 3 steht und Z für eine Gruppe der allgemeinen Formel -OR_{f'}T steht, worin R_{f'} für eine Fluorpolyoxyalkenkette mit einer Zahl von Wiederholungseinheiten von 0 bis 10 steht, wobei die Wiederholungseinheiten aus den folgenden: -CFXO-, -CF₂CFXO-, -CF₂CF₂CF₂O-, -CF₂CF₂CF₂CF₂O-ausgewählt sind, wobei X unabhängig für F oder CF₃ steht, und T für eine C₁-C₃-Perfluoralkylgruppe steht;
ausgewählt sind.

4. Verfahren nach Anspruch 3, wobei das Peroxid (P) der nachstehenden Formel (I) entspricht :
A-O-(CFXO)_{c1}(CF₂CF₂O)_{c2}(C₃F₆O)_{c3}(CF₂CF₂CF₂CF₂O)_{c4}(O)ₚ-B Formel (I)
worin :
- A und B gleich oder voneinander verschieden sind und unabhängig aus -CF₃, -CF₂-CF₃, -CF₂Cl, -CF₂CF₂Cl, -CF(CF₃)COF, -CF₂-COF, -COF ausgewählt sind;
- X für -F oder -CF₃ stehen;
- c1, c2, c3 und c4 gleich oder voneinander verschieden sind und unabhängig für ganze Zahlen ≥ 0 stehen und p für eine ganze Zahl > 0 steht, wobei c1, c2, c3, c4 und p so gewählt sind, dass das zahlenmittlere Molekulargewicht des Peroxids im Bereich von 300 bis 150.000 liegt und p so gewählt ist, dass der Gehalt an Peroxidgruppen -O-O- oder die Oxidationskraft (Oxidizing Power, OP) zwischen 0,02 % und 4 % liegt.

5. Verfahren nach einem der vorhergehenden Ansprüche, das unter kontinuierlichen, halbkontinuierlichen oder diskontinuierlichen Bedingungen durchgeführt wird.

6. Verfahren nach einem der vorhergehenden Ansprüche, das bei einer Temperatur im Bereich von -80°C bis 300°C durchgeführt wird.

7. Verfahren nach Anspruch 6, das bei einer Temperatur im Bereich von -80°C bis 150°C und unter UV-Katalyse durchgeführt wird.

8. Verfahren nach einem der vorhergehenden Ansprüche, das in Substanz oder in Gegenwart eines Lösungsmittels durchgeführt wird.

## Revendications

1. Procédé de fabrication de composés de (per)fluoropolyéther comportant deux extrémités de chaîne, où l'une des chaînes, ou les deux, se terminent par un groupement -CF₂- lié de façon covalente à un groupement aromatique ou hétéroaromatique éventuellement substitué via une liaison -carbone-carbone-,
ledit procédé comprenant la réaction d'un (per)fluoropolyéther peroxyde [peroxyde (P)] avec un composé aromatique ou hétéroaromatique éventuellement substitué, où
ledit composé aromatique est le suivant : benzène, biphényle, phénantrène, anthracène, acide benzoïque, fluorobenzène, bromobenzène, trifluorométhylbenzène, nitrobenzène, benzoate de méthyle ou d'éthyle, téréphtalate de diméthyle et pirocathécol ;
ledit composé aromatique ou hétéroaromatique pouvant porter un ou plusieurs substituants, qui peuvent être identiques ou différents les uns des autres, lesdits substituants sur le composé aromatique étant les suivants : halogène, (per)halogénoalkyle, nitro, amino, amido, nitrile, groupements esters et groupements phosphorés et soufrés.

2. Procédé selon la revendication 1 où le (per)fluoropolyéther peroxyde [peroxyde (P)] comprend au moins une entité peroxydique comprise entre des atomes de carbone sp³ et au moins une chaîne polyalkylène entièrement ou partiellement fluorée (chaîne Rf), c'est-à-dire un segment fluorocarboné comprenant des liaisons éther dans la chaîne principale.

3. Procédé selon la revendication 2, où la chaîne Rf comprend des motifs de répétition R°, distribués statistiquement le long de la chaîne, ledit motif de répétition étant choisi dans le groupe constitué par :
(i) (CFXO), où X représente F ou CF₃ ;
(ii) (CF₂CF₂O) ;
(iii) motifs (C₃F₆O) de formule (CF₂CF₂CF₂O), (CF₂CF(CF₃)O) et (CF(CF₃)CF₂O) ;
(iv) (CF₂CF₂CF₂CF₂O) ;
(v) (CR₅R₆CF₂CF₂O), où R₅ et R₆, qui peuvent être identiques ou différents, sont choisis parmi H, Cl et les groupements perfluoroalkyle comportant entre 1 et 4 atomes de carbone ;
(vi) -(CF₂)ⱼ-CFZ-O- où j représente un entier compris entre 0 et 3 et Z représente un groupement de formule générale -OR_{f}'T, où R_{f}' représente une chaîne fluoropolyoxyalcène comprenant un nombre de motifs de répétition compris entre 0 et 10, lesdits motifs de répétition étant choisis parmi les suivants : -CFXO-, -CF₂CFXO-, -CF₂CF₂CF₂O-, - CF₂CF₂CF₂CF₂O-, où X représente indépendamment F ou CF₃ et T représente un groupement perfluoroalkyle en C₁-C₃.

4. Procédé selon la revendication 3 dans lequel le peroxyde (P) est conforme à la formule (I) ci-après :
A-O-(CFXO)_{c1}(CF₂CF₂O)_{c2}(C₃F₆O)_{c3}(CF₂CF₂CF₂CF₂O)_{c4}(O)ₚ-B formule (I)
où :
- A et B sont identiques ou différents l'un de l'autre et sont indépendamment choisis parmi -CF₃, -CF₂-CF₃, -CF₂Cl, -CF₂CF₂Cl, -CF(CF₃)COF, -CF₂-COF, -COF ;
- X représente F ou CF₃ ;
- les nombres c1, c2, c3, et c4, égaux ou différents les uns des autres, représentent indépendamment des entiers ≥ 0 et p représente un entier > 0, où c1, c2, c3, c4 et p sont choisis de sorte que la masse moléculaire moyenne en nombre du peroxyde soit comprise entre 300 et 150 000 et p est choisi de sorte que la proportion de groupements peroxyde -O-O,- ou puissance oxydante (PO), soit comprise entre 0,02 % et 4 %.

5. Procédé selon l'une quelconque des revendications précédentes qui est mis en oeuvre dans des conditions continues, semi-continues ou discontinues.

6. Procédé selon l'une quelconque des revendications précédentes qui est mis en oeuvre à une température comprise entre -80°C et 300°C.

7. Procédé selon la revendication 6, qui est mis en oeuvre à une température comprise entre -80°C et 150°C et sous catalyse UV.

8. Procédé selon l'une quelconque des revendications précédentes, qui est mis en oeuvre en masse ou en présence d'un solvant.
